(19) Europäisches Patentamt
European Patent Office
Office européen des brevets

(11) Numéro de publication : **0 056 763**
**B1**

(12) **FASCICULE DE BREVET EUROPÉEN**

(45) Date de publication du fascicule du brevet : **04.07.84**

(51) Int. Cl.$^3$ : **C 07 D215/22**

(21) Numéro de dépôt : **82400077.2**

(22) Date de dépôt : **15.01.82**

(54) **Procédé de préparation de quinolinones-4.**

(30) Priorité : **16.01.81 FR 8100762**

(43) Date de publication de la demande : **28.07.82 Bulletin 82/30**

(45) Mention de la délivrance du brevet : **04.07.84 Bulletin 84/27**

(84) Etats contractants désignés : **AT BE CH DE FR GB IT LI LU NL SE**

(56) Documents cités :
**FR-A- 806 715**
**GB-A- 1 077 974**

(73) Titulaire : **RHONE-POULENC SANTE**
**Les Miroirs 18 Avenue d'Alsace**
**F-92400 Courbevole Cedex (FR)**

(72) Inventeur : **Baudouin, Michel**
**78, avenue Parmentier**
**F-69190 St Fons (FR)**
Inventeur : **Desbois, Michel**
**526, Chemin du Bois**
**F-69140 Rillieux la Pape (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

EP 0 056 763 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un nouveau procédé de préparation de tétrahydro-1,2,3,4 quinolinones-4 de formule générale :

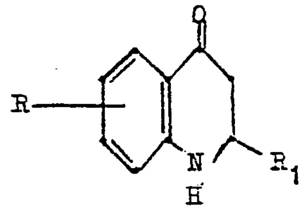

(I)

qui sont particulièrement utiles comme intermédiaires dans la synthèse de substances thérapeutiquement actives.

Dans la formule générale (I), R représente un atome d'hydrogène ou un substituant choisi dans le groupe des atomes d'halogène et des radicaux alcoyles droits ou ramifiés contenant 1 à 4 atomes de carbone alcoyloxy droits ou ramifiés contenant 1 à 4 atomes de carbone et trifluorométhyle, et $R_1$ représente un atome d'hydrogène ou un radical alcoyle contenant 1 à 4 atomes de carbone ou trifluorométhyle.

Il est connu de préparer des tétrahydro-1,2,3,4 quinolinones-4 de formule générale (I) par cyclisation d'un acide anilino-3 propionique dont la fonction amine est éventuellement substituée au moyen d'acide sulfurique, d'anhydride phosphorique, d'acide phosphorique ou polyphosphorique.

Il est connu également, par exemple d'après le brevet français 806 715 de cycliser un arylamino-3 propionitrile substitué ou non à l'azote en opérant en présence de chlorure d'aluminium, la cyclisation pouvant être effectuée en utilisant d'autres agents tels que le trifluorure de bore, le bromure d'aluminium, les halogénures de titane, étain, arsenic, antimoine ou les oxyhalogénures de phosphore ($POCl_3$) ou le chlorure ferrique ainsi que les hydracides halogénés ou l'acide sulfurique.

Il a maintenant été trouvé, et c'est ce qui fait l'objet de la présente invention, que les tétrahydro-1,2,3,4 quinolinones-4 de formule générale (I) peuvent être obtenues avec des rendements pratiquement quantitatifs par cyclisation d'un acide anilino-3 propionique de formule générale :

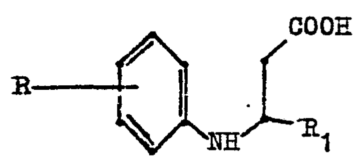

(II)

dans laquelle R et $R_1$ sont définis comme précédemment, en présence d'un mélange d'acide fluorhydrique et de trifluorure de bore.

Ce résultat est d'autant plus surprenant et inattendu que la cyclisation d'un acide de formule générale (II) ne se produit pas en présence d'acide fluorhydrique seul ou de trifluorure de bore seul et que l'anilino-3 propionitrile correspondant en présence d'un mélange d'acide fluorhydrique et de trifluorure de bore ne conduit pas à la quinolinone-4.

Pour la mise en œuvre du procédé selon la présente invention, il est particulièrement avantageux de chauffer une solution d'un acide de formule générale (II) dans l'acide fluorhydrique saturée de trifluorure de bore, de préférence sous pression.

L'acide de formule générale (II) peut être dissous dans l'acide fluorhydrique à raison de 1 mole d'acide de formule générale (II) pour 15 à 50 moles d'acide fluorhydrique.

Pour obtenir une conversion complète de l'acide de formule générale (II), il est nécessaire d'engager dans la réaction au moins 2 moles de trifluorure de bore par mole d'acide de formule générale (II). Le trifluorure de bore peut être engagé soit dans sa totalité au début de la réaction soit progressivement au cours de la réaction de manière à maintenir une pression partielle comprise entre 1 et 30 bars.

Lorsque l'on met en œuvre un acide de formule générale (II) dans laquelle le substituant R est en position méta par rapport à la fonction amine et représente un atome d'halogène ou un radical alcoyle ou alcoyloxyle, on obtient la tétrahydro-1,2,3,4 quinolinone-4 de formule générale (I) substituée en position −7 pratiquement exempte de l'isomère substitué en position −5 qu'il est théoriquement possible d'obtenir.

Lorsque l'acide de formule générale (II) est l'acide m-chloroanilino-3 propionique, les résultats sont particulièrement satisfaisants. En effet, alors que, selon le brevet français 1 514 280, la cyclisation de l'acide m-chloroanilino-3 propionique, au moyen d'acide polyphosphorique à une température voisine de 100 °C, conduit à un mélange, en proportions pratiquement égales, de chloro-5 tétrahydro-1,2,3,4

quinolinone-4 et de chloro-7 tétrahydro-1,2,3,4 quinolinone-4 dont les constituants doivent être séparés en vue de leur mise en œuvre ultérieure, le procédé selon l'invention permet d'obtenir la chloro-7 tétrahydro-1,2,3,4 quinolinone-4 avec une teneur en isomère chloro-5 qui peut être inférieure à 1 %.

A cet effet, les meilleurs résultats sont obtenus lorsque l'on effectue la cyclisation dans un mélange réactionnel constitué par une solution de l'acide m-chloroanilino-3 propionique dans l'acide fluorhydrique, à raison d'une molécule d'acide m-chloroanilino-3 propionique pour 15 à 50 moles d'acide fluorhydrique, saturée à 20 °C, de trifluorure de bore sous une pression comprise entre 2 et 16 bars de préférence voisine de 12 bars et à une température comprise entre 60 et 100 °C, de préférence voisine de 80 °C pendant environ 20 heures.

Dans ces conditions, le taux de transformation est voisin de 100 % et la teneur en chloro-5 tétrahydro-1,2,3,4 quinolinone-4 est inférieure à 1 %.

La tétrahydro-1,2,3,4 quinolinone-4 de formule générale (I) obtenue selon le procédé de la présente invention peut être isolée du mélange réactionnel selon les méthodes habituelles. Cependant il est particulièrement avantageux de pouvoir recycler l'acide fluorhydrique et le trifluorure de bore mis en œuvre. A cet effet, il est possible d'opérer de la manière suivante :

— la réaction terminée, le mélange réactionnel peut être refroidi puis dégazé ce qui permet de récupérer la majeure partie du trifluorure de bore non combiné aux produits organiques, puis

— l'acide fluorhydrique est distillé, éventuellement après addition d'un solvant organique tel que le chlorobenzène ou le tétrachlorure de carbone ; dans ces conditions, la quinolinone salifiée partiellement par l'acide tétrafluoroborique est en suspension dans le solvant, et enfin

— la quinolinone est déplacée de son sel par addition d'une base, telle que l'ammoniac, et est obtenue en solution dans le solvant, le sel insoluble de l'acide tétrafluoroborique et de la base étant séparé par filtration.

La tétrahydro-1,2,3,4 quinolinone-4 de formule générale (I) est isolée après concentration éventuelle de sa solution et est séparée par filtration ou décantation.

La tétrahydro-1,2,3,4 quinolinone-4 de formule générale (I) peut être purifiée par application des méthodes habituelles telles que la cristallisation ou la chromatographie.

Les acides de formule générale (II) utilisés comme produit de départ peuvent être obtenus par action d'une aniline convenablement substituée sur un acide de formule générale :

$$R_1—CH = CH—COOH \qquad (III)$$

dans laquelle $R_1$ est défini comme précédemment.

La réaction s'effectue généralement dans l'eau à une température comprise entre 70 et 100 °C en utilisant un excès d'aniline par rapport à l'acide de formule générale (III) mis en œuvre. La durée de la réaction est généralement comprise entre 1 et 4 heures.

Les tétrahydro-1,2,3,4 quinolinones-4 de formule générale (I) sont particulièrement utiles comme intermédiaires dans la synthèse de substances thérapeutiquement actives telles que la chloroquine, la glafénine, l'antrafénine ou l'amodiaquine.

Plus particulièrement, la chloro-7 tétrahydro-1,2,3,4 quinolinone-4 peut être transformée en chloroquine par condensation de la diéthylamino-4 méthyl-1 butylamine en présence d'air selon le procédé décrit par W. S. JOHNSON et B. G. BUELL, J. Amer. Chem. Soc., *74,* 4513 (1952).

Les exemples suivants montrent comment l'invention peut être mise en pratique.

## Exemple 1

Dans un réacteur en acier inoxydable contenant 50 g d'acide fluorhydrique liquide refroidi à 5 °C on ajoute 10 g d'acide m-chloroanilino-3 propionique (titrant 94,5 %). La solution est saturée de trifluorure de bore gazeux. A cet effet le contenu du réacteur est maintenu à 20 °C puis saturé par le trifluorure de bore gazeux sous une pression de 12 bars pendant 1 heure. Le réacteur est ensuite fermé puis chauffé à 80 °C pendant 20 heures.

Au cours du chauffage la pression s'élève d'abord jusqu'à 20 bars puis diminue progressivement pour se stabiliser vers 16 bars. Le réacteur est ensuite refroidi à 10 °C puis ouvert de manière à laisser échapper le trifluorure de bore. Le liquide rougeâtre obtenu est versé dans un mélange d'eau et de glace. Après extraction 3 fois par 100 $cm^3$ de chloroforme, la couche organique est lavée plusieurs fois par 100 $cm^3$ d'eau jusqu'à un pH compris entre 3 et 4 puis séchée sur sulfate de sodium. Après filtration et concentration à sec sous pression réduite (10 mm de mercure ; 1,33 kPa), on obtient 9 g de chloro-7 tétrahydro-1,2,3,4 quinolinone-4 cristallisée dont le titre déterminé par chromatographie en phase gazeuse est de 94,5 %.

Le taux de transformation est de 100 % et le rendement par rapport à l'acide m-chloroanilino-3 propionique est de 99 %.

Par examen en chromatographie en phase gazeuse, on détermine que la teneur en isomère chloro-5 est voisine de 0,7 %.

L'acide m-chloroanilino-3 propionique de départ peut être préparé de la manière suivante :

A un mélange de 510,3 g de m-chloroaniline dans 150 $cm^3$ d'eau, maintenu sous atmosphère d'argon

agité à 80 °C, on ajoute en 10 minutes une solution de 72,05 g d'acide acrylique dans 100 $cm^3$ d'eau. Le mélange réactionnel, constitué de deux phases, est maintenu pendant 3 heures à 80 °C sous agitation puis refroidi à 20 °C. Après décantation, la phase aqueuse (couche supérieure) est éliminée. A la phase organique on ajoute 423 $cm^3$ d'une solution aqueuse de soude 2,6 N, en agitant et en maintenant la température à 20 °C. Après décantation, la phase organique constituée de 303 g de m-chloroaniline est séparée. La phase aqueuse (850 $cm^3$) est extraite 6 fois successivement par 450 $cm^3$ d'éther.

La phase aqueuse, dont on élimine l'éther par évaporation sous pression réduite (20 mm de mercure ; 2,7 kPa), est acidifiée par addition de 105 g d'acide sulfurique à 50 % (en poids). Le pH final est 3,5 (point isoélectrique). La température passe de 22 à 33 °C puis on chauffe à 40 °C. Après décantation, on sépare :

— une phase organique inférieure (208,8 g) constituée d'acide m-chloroanilino-3 propionique fondu saturé d'eau (8,6 % d'eau)

— une phase aqueuse supérieure (601 g) contenant 2,28 g d'acide m-chloroanilino-3 propionique et 156 g de sulfate de sodium.

La phase organique est chauffée pendant 1 heure à 80 °C sous pression réduite (20 mm de mercure ; 2,7 kPa). On obtient 195,4 g d'un produit contenant 94 % d'acide m-chloroanilino-3 propionique et 2,3 % d'eau.

Exemple 2

Dans un réacteur en acier inoxydable contenant 100 g d'acide fluorhydrique liquide refroidi à 5 °C, on ajoute 23,3 g d'acide o-trifluorométhylanilino-3 propionique. La solution est saturée de trifluorure de bore gazeux. A cet effet, le contenu du réacteur est maintenu à 20 °C puis saturé par le trifluorure de bore gazeux sous une pression de 17 bars pendant 1 heure. Le réacteur est ensuite fermé puis chauffé à 80 °C pendant 19 heures.

Le mélange réactionnel est alors traité dans les conditions de l'exemple 1. On obtient ainsi 13,9 g de trifluorométhyl-8 tétrahydro-1,2,3,4 quinolinone-4 dont le titre, déterminé par chromatographie en phase gazeuse, est de 99 %.

Le taux de transformation est de 68,7 % et le rendement par rapport à l'acide o-trifluorométhylanilino-3 propionique transformé est voisin de 95 %.

Exemple 3

Dans un réacteur en acier inoxydable contenant 100 g d'acide fluorhydrique liquide refroidi à 5 °C, on ajoute 21,4 g d'acide m-chloroanilino-3 butanoïque. La solution est saturée de trifluorure de bore gazeux. A cet effet, le contenu du réacteur est maintenu à 20 °C puis saturé de trifluorure de bore gazeux sous une pression de 10 bars pendant 1 heure. Le réacteur est ensuite fermé puis chauffé à 82 °C pendant 24 heures.

Le mélange réactionnel est alors traité dans les conditions de l'exemple 1. On obtient ainsi 18,3 g de chloro-7 méthyl-2 tétrahydro-1,2,3,4 quinolinone-4 dont le titre, déterminé par chromatographie en phase gazeuse, est de 95 %.

Le taux de transformation est de 93,5 % et le rendement par rapport à l'acide m-chloroanilino-3 butanoïque transformé est voisin de 100 %.

Par examen en chromatographie en phase gazeuse et en résonnance magnétique nucléaire, la teneur en isomère chloro-5 est inférieure à 1 %.

Exemple 4

Dans un réacteur en acier inoxydable contenant 100 g d'acide fluorhydrique liquide refroidi à 5 °C, on ajoute 18,5 g d'acide m-méthylanilino-3 propionique. La solution est saturée de trifluorure de bore gazeux. A cet effet, le contenu du réacteur est maintenu à 20 °C puis saturé de trifluorure de bore gazeux sous une pression de 11 bars pendant 1 heure. Le réacteur est ensuite chauffé à 82 °C pendant 23 heures.

Le mélange réactionnel est alors traité dans les conditions de l'exemple 1. On obtient ainsi 16,6 g de méthyl-7 tétrahydro-1,2,3,4 quinolinone-4 dont le titre, déterminé par chromatographie en phase gazeuse, est de 96 %.

Le taux de transformation est voisin de 100 % et le rendement par rapport à l'acide m-méthylanilino-3 propionique transformé est voisin de 100 %.

Par examen en chromatographie en phase gazeuse et en résonance magnétique nucléaire, la teneur en isomère méthyl-5 est inférieure à 1 %.

**Revendications**

1. Procédé de préparation d'une tétrahydro-1,2,3,4 quinolinone-4 de formule générale :

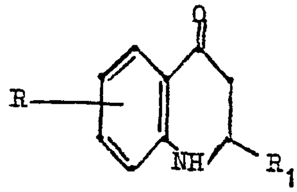

dans laquelle R représente un atome d'hydrogène ou un substituant choisi dans le groupe des atomes d'halogène et des radicaux alcoyles droits ou ramifiés contenant 1 à 4 atomes de carbone, alcoyloxy droits ou ramifiés contenant 1 à 4 atomes de carbone et trifluorométhyle et $R_1$ représente un atome d'hydrogène ou un radical alcoyle contenant 1 à 4 atomes de carbone ou trifluorométhyle, caractérisé en ce que l'on cyclise un acide anilino-3 propionique de formule générale :

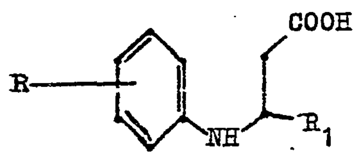

dans laquelle R et $R_1$ sont définis comme précédemment, en solution dans un mélange contenant au moins 2 moles de trifluorure de bore et 15 à 50 moles d'acide fluorhydrique par mole d'acide anilino-3 propionique, en opérant à une température comprise entre 20 et 120 °C et sous une pression comprise entre 1 et 30 bars.

2. Procédé selon la revendication 1 pour la préparation d'une tétrahydro-1,2,3,4 quinolinone-4 de formule générale :

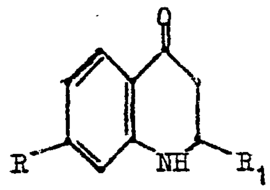

dans laquelle R représente un atome d'halogène ou un radical alcoyle droit ou ramifié contenant 1 à 4 atomes de carbone ou alcoyloxy droit ou ramifié contenant 1 à 4 atomes de carbone et $R_1$ est défini comme dans la revendication 1, pratiquement exempte de l'isomère en position – 5, caractérisé en ce que l'on cyclise un acide anilino-3 propionique de formule générale :

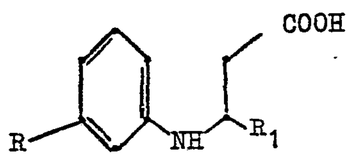

dans laquelle R et $R_1$ sont définis comme ci-dessus, en solution dans un mélange contenant au moins 2 moles de trifluorure de bore et 15 à 50 moles d'acide fluorhydrique par mole d'acide anilino-3 propionique en opérant à une température comprise entre 20 et 120 °C et sous une pression comprise entre 1 et 30 bars.

3. Procédé selon la revendication 1 pour la préparation de la chloro-7 tétrahydro-1,2,3,4 quinolinone-4 pratiquement exempte de chloro-5 tétrahydro-1,2,3,4 quinolinone-4 caractérisé en ce que l'on cyclise l'acide m-chloroaniline-3 propionique en solution dans l'acide fluorhydrique, à raison d'une mole d'acide m-chloroanilino-3 propionique pour 15 à 50 moles d'acide fluorhydrique, saturée de trifluorure de bore sous une pression comprise entre 1 et 30 bars et à une température comprise entre 20 et 100 °C.

4. Procédé selon la revendication 3 caractérisé en ce que la pression est comprise entre 2 et 16 bars et la température est comprise entre 70 et 90 °C.

5. Procédé selon la revendication 3 caractérisé en ce que l'on utilise au moins 2 moles de trifluorure de bore par mole d'acide m-chloroanilino-3 propionique.

**Claims**

1. Process for the preparation of a 1,2,3,4-tetrahydroquinolin-4-one of the general formula :

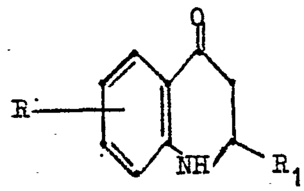

in which R represents a hydrogen atom or a substituent chosen from the group comprising halogen atoms and linear or branched alkyl radicals containing 1 to 4 carbon atoms, linear or branched alkoxy radicals containing 1 to 4 carbon atoms and trifluoromethyl radicals, and $R_1$ represents a hydrogen atom, an alkyl radical containing 1 to 4 carbon atoms or a trifluoromethyl radical, characterised in that a 3-anilinopropionic acid of the general formula :

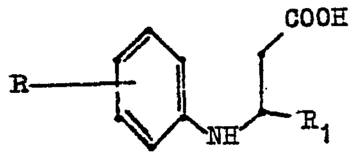

in which R and $R_1$ are defined as above, is cyclised in solution in a mixture containing at least 2 mol of boron trifluoride and 15 to 50 mol of hydrofluoric acid per mol of 3-anilinopropionic acid, the reaction being carried out at a temperature of between 20 and 120 °C and under a pressure of between 1 and 30 bar.

2. Process according to Claim 1 for the preparation of 1,2,3,4-tetrahydroquinolin-4-one of the general formula :

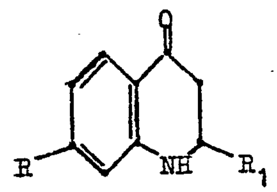

in which R represents a halogen atom, a linear or branched alkyl radical having 1 to 4 carbon atoms or a linear or branched alkoxy radical containing 1 to 4 carbon atoms, and $R_1$ is defined as in claim 1, and which is virtually free of the isomer in the 5-position, characterised in that a 3-anilinopropionic acid of the general formula :

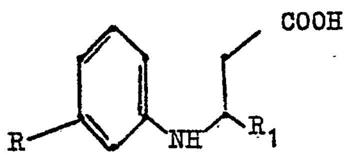

in which R and $R_1$ are defined as above, is cyclised in solution in a mixture containing at least 2 mol of boron trifluoride and 15 to 50 mol of hydrofluoric acid per mol of 3-anilinopropionic acid, the reaction being carried out at a temperature of between 20 and 120 °C and under a pressure of 1 and 30 bar.

3. Process according to Claim 1 for the preparation of 7-chloro-1,2,3,4-tetrahydroquinolin-4-one virtually free of 5-chloro-1,2,3,4-tetrahydroquinolin-4-one, characterised in that 3-m-chloroanilinopropionic acid is cyclised in solution in hydrofluoric acid, in proportions of one mol of 3-m-chloroanilinopropionic acid per 15 to 50 mol of hydrofluoric acid, the solution being saturated with boron trifluoride under a pressure of between 1 and 30 bar and a temperature of between 20 and 100 °C.

4. Process according to Claim 3, characterised in that the pressure is between 2 and 16 bar and the temperature is between 70 and 90 °C.

5. Process according to Claim 3, characterised in that at least 2 mol of boron trifluoride are used per mol of 3-m-chloroanilinopropionic acid.

**Ansprüche**

1. Verfahren zur Herstellung eines 1,2,3,4-Tetrahydrochinolin-4-ons der allgemeinen Formel :

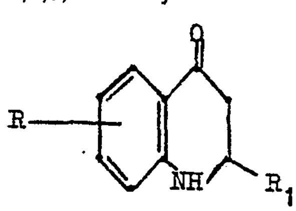

in welcher R ein Wasserstoffatom oder einen Substituenten, ausgewählt aus der Gruppe der Halogenatome und der geradkettigen oder verzweigten Alkylreste mit 1 bis 4 Kohlenstoffatomen, der geradkettigen oder verzweigten Alkyloxyreste mit 1 bis 4 Kohlenstoffatomen und dem Trifluormethylrest, darstellt und $R_1$ ein Wasserstoffatom oder einen Alkylrest mit 1 bis 4 Kohlenstoffatomen oder den Trifluormethylrest darstellt, dadurch gekennzeichnet, daß man ein 3-Anilinopropionsäure der allgemeinen Formel :

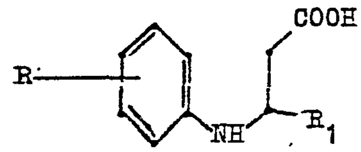

in welcher R und $R_1$ die obige Bedeutung haben, in Lösung in einer Mischung, die zumindest 2 Mol Bortrifluorid und 15 bis 50 Mol Fluorwasserstoffsäure pro Mol 3-Anilinopropionsäure enthält, cyclisiert, wobei man bei einer Temperatur von 20 bis 120 °C und unter einem Druck von 1 bis 30 bar arbeitet.

2. Verfahren nach Anspruch 1, zur Herstellung eines 1,2,3,4-Tetrahydro-chinolin-4-ons der allgemeinen Formel :

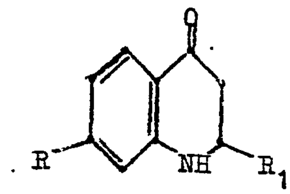

in welcher R ein Wasserstoffatom oder einen geradkettigen oder verzweigten Alkylrest mit 1 bis 4 Kohlenstoffatomen oder einen geradkettigen oder verzweigten Alkyloxyrest mit 1 bis 4 Kohlenstoffatomen darstellt und $R_1$ die im Anspruch 1 angegebene Bedeutung hat, das praktisch frei vom Isomeren in 5-Stellung ist, dadurch gekennzeichnet, daß man eine 3-Anilinopropionsäure der allgemeinen Formel :

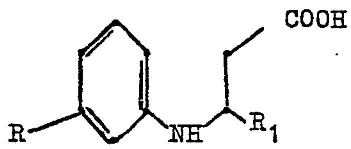

in welcher R und $R_1$ die obige Bedeutung haben, in Lösung in einer Mischung cyclisiert, die zumindest 2 Mol Bortrifluorid und 15 bis 50 Mol Fluorwasserstoffsäure pro Mol 3-Anilinopropionsäure enthält, wobei man bei einer Temperatur von 20 bis 120 °C und unter einem Druck von 1 bis 30 bar arbeitet.

3. Verfahren nach Anspruch 1 zur Herstellung von 7-Chlor-1,2,3,4-tetrahydro-chinolin-4-on, das praktisch frei von 5-Chlor-1,2,3,4-tetrahydro-chinolin-4-on ist, dadurch gekennzeichnet, daß man 3-m-Chloranilinopropionsäure, gelöst in Fluorwasserstoffsäure in einem Anteil von 1 Mol 3-m-Chloranilinopropionsäure pro 15 bis 50 Mol Fluorwasserstoffsäure, gesättigt mit Bortrifluorid, unter einem Druck von 1 bis 30 bar und bei einer Temperatur von 20 bis 100 °C cyclisiert.

4. Verfahren nach Anspruch 3, dadurch gekennzeichnet, daß der Druck 2 bis 16 bar und die Temperatur 70 bis 90 °C beträgt.

5. Verfahren nach Anspruch 3, dadurch gekennzeichnet, daß man zumindest 2 Mol Bortrifluorid pro Mol 3-m-Chloranilinopropionsäure einsetzt.